(19) Europäisches Patentamt
European Patent Office
Office européen des brevets

(11) EP 4 498 067 A1

# (12) EUROPEAN PATENT APPLICATION

(43) Date of publication:
**29.01.2025 Bulletin 2025/05**

(21) Application number: **24190093.5**

(22) Date of filing: **22.07.2024**

(51) International Patent Classification (IPC):
***G01N 21/3504*** *(2014.01)* ***G01N 21/39*** *(2006.01)*
*G01N 21/359* *(2014.01)*

(52) Cooperative Patent Classification (CPC):
**G01N 21/39;** G01N 21/3504; G01N 21/359;
G01N 2021/354; G01N 2021/399

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.07.2023 JP 2023123851**

(71) Applicant: **Yokogawa Electric Corporation**
**Tokyo 180-8750 (JP)**

(72) Inventors:
- **Murata, Akihiro**
  **Musashino-shi, 180-8750 (JP)**
- **Takeda, Shoma**
  **Musashino-shi, 180-8750 (JP)**

(74) Representative: **Zimmermann, Tankred Klaus**
**Schoppe, Zimmermann, Stöckeler**
**Zinkler, Schenk & Partner mbB**
**Patentanwälte**
**Radlkoferstrasse 2**
**81373 München (DE)**

## (54) MEASUREMENT APPARATUS AND MEASUREMENT METHOD

(57) A measurement apparatus (1) includes a vaporization chamber (21) in which a sample (S) containing a target component is placed, a measurement chamber (23) that is spatially connected to the vaporization chamber (21) and forms enclosed space integrally with the vaporization chamber (21), a light source (31) that applies, to the measurement chamber (23), laser light whose wavelength varies, a detector (51) that detects the laser light having been applied from the light source (31) and having passed through the measurement chamber (23), and an arithmetic unit (60) that calculates the amount of the target component contained in the sample having been vaporized by heating in the vaporization chamber (21) by analyzing a light-receiving signal of the laser light detected by the detector (51). The arithmetic unit (60) acquires the amount of the target component, based on an integral value of a spectrum of absorbance of the laser light that is absorbed by the target component while the laser light passes through an interior of the measurement chamber (23).

*FIG. 6*

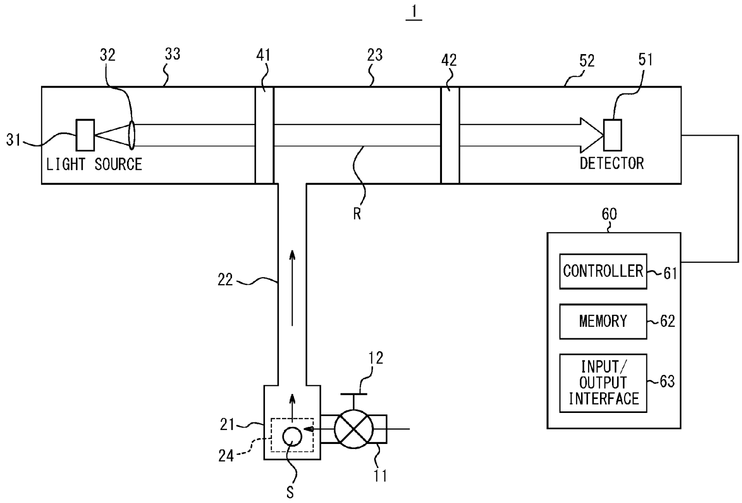


EP 4 498 067 A1

## Description

CROSS-REFERENCE TO RELATED APPLICATION

This application claims priority to Japanese Patent Application No. 2023-123851, filed on July 28, 2023, the entire contents of which are incorporated herein by reference.

TECHNICAL FIELD

The present disclosure relates to a measurement apparatus and a measurement method.

BACKGROUND

With the advancement of space development technologies, technology for measuring the contents, concentrations, or the like of water and other volatile components in regolith or other samples on the lunar surface is known. For example, it is known to measure water or another volatile component by collecting the regolith on the lunar surface, heating the regolith to vaporize the component to be measured, and measuring the vaporized component (Non-Patent Literature (NPL) 1). The lunar surface is often in cryogenic conditions, and water in the regolith often exists as a solid (ice). The water that exists as the solid in the cryogenic conditions may sublimate in response to heating. Therefore, in this document, the term "vaporization" includes both sublimation, which is change from solid to gas, and evaporation, which is change from liquid to gas.

CITATION LIST

Non-patent Literature

NPL 1: SHIMOMURA Takuma and 12 others, "Lunar Polar Exploration Mission: Study on water resource observation", Proceedings of the 66th Space Sciences and Technology Conference, The Japan Society for Aeronautical and Space Sciences, 2022

SUMMARY

However, there is room for improvement in measurement accuracy in conventional technology for measuring volatile components.

It would be helpful to enable measurement of a volatile component with higher accuracy.

(1) A measurement apparatus according to some embodiments includes:

a vaporization chamber in which a sample containing a target component is placed;
a measurement chamber spatially connected to the vaporization chamber, the measurement chamber configured to form enclosed space integrally with the vaporization chamber;
a light source configured to apply, to the measurement chamber, laser light whose wavelength varies;
a detector configured to detect the laser light that has been applied from the light source and has passed through the measurement chamber; and
an arithmetic unit configured to calculate the amount of the target component contained in the sample by analyzing a light-receiving signal of the laser light detected by the detector,
wherein the arithmetic unit is configured to acquire the amount of the target component, based on an integral value of a spectrum of absorbance of the laser light that is absorbed by the target component while the laser light passes through an interior of the measurement chamber.

The measurement apparatus acquires the amount of the target component based on the integral value of the spectrum of the absorbance obtained from the intensity of the laser light absorbed by the target component while the laser light passes through the interior of the measurement chamber. Therefore, the amount of the target component can be measured with high accuracy even when pressure in the enclosed space fluctuates significantly.

In one embodiment,
(2) in the measurement apparatus according to (1), the arithmetic unit may be configured to acquire curve data by performing curve fitting of the spectrum of the absorbance of the laser light to a model equation, and acquire the integral value of the spectrum based on the curve data.

The measurement apparatus acquires the integral value (area) of the spectrum by performing the curve fitting of the spectrum of the absorbance of the laser light to the model equation. Therefore, the spectrum of the absorbance for the target component can be measured with higher accuracy.

In one embodiment,
(3) in the measurement apparatus according to (1) or (2), the light source may be a wavelength tunable semiconductor laser configured to be able to vary the wavelength of the laser light to be output.

With the use of the wavelength tunable semiconductor laser as the light source, the measurement apparatus can measure the amount of the target component based on the integral value of the absorbance by wavelength sweep over a range that includes an absorption line center wavelength of the target component.

In one embodiment,
(4) in the measurement apparatus according to any one of (1) to (3),

the light source may be configured to apply, as the laser light, light whose wavelength varies periodically, and
the arithmetic unit may be configured to acquire the spectrum for each of a plurality of periods with which the wavelength of the laser light varies, and acquire the amount of the target component based on a plurality of the spectra.

The measurement apparatus acquires the spectrum for each of the plurality of periods with which the wavelength of the laser light varies, and acquires the amount of the target component based on the plurality of the spectra. Therefore, the measurement apparatus can measure the amount of the target component with even higher accuracy with reduced influence of variations in data from period to period.

In one embodiment,

(5) the measurement apparatus according to any one of (1) to (4), may further include:

a heater configured to control the temperature of the sample,

wherein the arithmetic unit may be configured to output relational data indicating a correspondence between the temperature of the sample and the amount of the target component calculated by analyzing the light-receiving signal of the laser light.

The measurement apparatus outputs the relational data indicating the correspondence between the temperature of the sample and the amount of the target component. Therefore, the measurement apparatus can analyze, based on the relational data, a binding state of the target component in the sample.

(6) A measurement method according to some embodiments includes:

applying, by a light source to a measurement chamber, laser light whose wavelength varies, the measurement chamber being spatially connected to a vaporization chamber in which a sample containing a target component is placed, the measurement chamber configured to form enclosed space integrally with the vaporization chamber;

detecting, by a detector, the laser light that has been applied from the light source and has passed through the measurement chamber; and

calculating, by an arithmetic unit, the amount of the target component contained in the sample by analyzing a light-receiving signal of the laser light detected by the detector,

wherein the arithmetic unit is configured to acquire the amount of the target component, based on an integral value of a spectrum of absorbance of the laser light that is absorbed by the target component while the laser light passes through an interior of the measurement chamber.

The measurement apparatus acquires the amount of the target component based on the integral value of the spectrum of the absorbance obtained from the intensity of the laser light that is absorbed by the target component while the laser light passes through the interior of the measurement chamber. Therefore, the amount of the target component can be measured with high accuracy even when pressure in the enclosed space fluctuates significantly.

In one embodiment,

(7) in the measurement method according to (6), the arithmetic unit may be configured to acquire curve data by performing curve fitting of the spectrum of the absorbance of the laser light to a model equation, and acquire the integral value of the spectrum based on the curve data.

The measurement apparatus acquires the integral value (area) of the spectrum by performing the curve fitting of the spectrum of the absorbance of the laser light to the model equation. Therefore, the spectrum of the absorbance for the target component can be measured with higher accuracy.

In one embodiment,

(8) in the measurement method according to (6) or (7), the light source may be a wavelength tunable semiconductor laser configured to be able to vary the wavelength of the laser light to be output.

With the use of the wavelength tunable semiconductor laser as the light source, the measurement apparatus can measure the amount of the target component based on the integral value of the spectrum by wavelength sweep over a range that includes an absorption line center wavelength of the target component.

In one embodiment,

(9) in the measurement method according to any one of (6) to (8),

the light source may be configured to apply, as the laser light, light whose wavelength varies periodically, and

the arithmetic unit may be configured to acquire the spectrum for each of a plurality of periods with which the wavelength of the laser light varies, and acquire the amount of the target component based on a plurality of the spectra.

The measurement apparatus acquires the spectrum for each of the plurality of periods with which the wavelength of the laser light varies, and acquires the amount of the target component based on the plurality of the spectra. Therefore, the measurement apparatus can measure the amount of the target component with even higher accuracy with reduced influence of variations in data from period to period.

In one embodiment,

(10) the measurement method according to any one of (6) to (9), may further include:

controlling, by a heater, the temperature of the sample,

wherein the arithmetic unit may be configured to output relational data indicating a correspondence between the temperature of the sample and the amount of the target component calculated by analyzing the light-receiving signal of the laser light.

The measurement apparatus outputs the relational data indicating the correspondence between the temperature of the sample and the amount of the target component. Therefore, the measurement apparatus can analyze, based on the relational data, a binding state of the target component in the sample.

According to an embodiment of the present disclosure, a volatile component can be measured with higher accuracy.

BRIEF DESCRIPTION OF THE DRAWINGS

In the accompanying drawings:

FIG. 1 is a diagram illustrating a configuration of a measurement apparatus according to a comparative example;
FIG. 2 is a diagram illustrating an example of a graph indicating the relationship between the concentration of water and internal pressure in a case in which water is an only gaseous component;
FIG. 3 is a diagram illustrating an example of a graph indicating variations of a spectrum of absorbance for water vapor with pressure;
FIG. 4 is a diagram illustrating a portion of the graph in FIG. 3 with enlargement;
FIG. 5 is a diagram illustrating an example of a graph indicating the relationship between the height of the spectrum of the absorbance and the amount of water;
FIG. 6 is a diagram illustrating an example of a configuration of a measurement apparatus according to an embodiment;
FIG. 7 is a flowchart illustrating an example of operations of the measurement apparatus in FIG. 6;
FIG. 8 is a diagram schematically illustrating variation of laser drive current with time;
FIG. 9 is a diagram schematically illustrating a graph indicating the relationship between the laser drive current and wavelength;
FIG. 10 is a diagram schematically illustrating variation, with time, of the intensity of a light-receiving signal detected by a detector;
FIG. 11 is a diagram schematically illustrating the relationship between laser wavelength and the intensity of the light-receiving signal;
FIG. 12 is a diagram schematically illustrating the relationship between the laser wavelength and the intensity of the light-receiving signal processed by baseline correction;
FIG. 13 is a diagram schematically illustrating the relationship between the laser wavelength and absorbance;
FIG. 14 is a diagram schematically illustrating the relationship between the height and area of the spectrum of the absorbance and the amount of a target component; and
FIG. 15 is a diagram illustrating an example of relational data indicating the relationship between temperature and the amount of the target component.

DETAILED DESCRIPTION

<Comparative Example>

FIG. 1 is a diagram illustrating a configuration of a measurement apparatus 9 according to a comparative example. The measurement apparatus 9 is an apparatus that measures the amount of a target component, such as water, contained in a solid sample, such as regolith. The measurement apparatus 9 seals the sample in enclosed space, and heats the sample to vaporize the target component contained in the sample. The measurement apparatus 9 applies, to the volatile gas, laser light with a single wavelength selected according to a wavelength to be absorbed by the target component. The measurement apparatus 9 measures absorbance for the volatile gas, and measures the amount of the target component based on the height (value) of the absorbance.

The measurement apparatus 9 is provided with a light source 91, a detector 92, and a measurement chamber 93. The measurement chamber 93 provides the enclosed space in which a gas 94 containing the target component vaporized from the sample exists. The light source 91 applies the laser light (incident light) to the measurement chamber 93. The detector 92 detects the laser light (transmitted light) that has been applied from the light source 91 and has passed through the measurement chamber 93. The measurement apparatus 9 is envisioned to be used in an environment in which there is no atmosphere, such as the lunar surface. Even in the measurement chamber 93, there are no gas components other than the gas 94 vaporized from the sample.

In the configuration illustrated in FIG. 1, after introducing the sample to be measured, such as the regolith, into the measurement chamber 93, the measurement apparatus 9 heats the sample to vaporize the target component from the sample after making the chamber 93 into the enclosed space. An example in which the target component is water will be hereinafter described. In the presence of the gas vaporized from the target component, the light source 91 outputs the laser light with the specific single wavelength that is known to be absorbed by the target component. The measurement apparatus 9 calculates the ratio of the intensity $I_0$ of the laser light output from the light source 91 and the intensity It of the laser light passing through the measurement chamber 93 and detected by the detector 92, to acquire the absorbance $H = \ln(I_0/I_t)$. Here, ln is a logarithmic function with natural logarithm e as the base.

In such a case, it is known that the concentration C of the gas of the target component is proportional to the absorbance H and inversely proportional to an optical path length L (Lambert-Beer's law). The optical path length L is a length through which the laser light passes across space in which the gas 94 exists in the measurement chamber 93. The Lambert-Beer's law is expressed by the following equation 1, in which ε represents an absorption coefficient intrinsic to the molecules of the target component.

(Equation 1)

$$C = H/\varepsilon L = \ln(I_0/I_t)/\varepsilon L$$

Therefore, by measuring the optical path length L in advance in the apparatus, and by measuring a correspondence between an absorbance value measured by introducing a known concentration of the gas of the target component and the concentration to acquire the absorption coefficient ε in advance, the measurement apparatus 9 can measure the concentration of the gas from the measured absorbance. When the mass of the regolith or another sample fed here is known, the measurement apparatus 9 can calculate the concentration of water in the regolith.

However, as the concentration of the target component increases, pressure in the measurement chamber 93 increases, and accordingly the absorbance is no longer exactly proportional to the concentration. Therefore, such an increase in pressure may cause an error in a measurement value of the target component. The influence of the pressure in the measurement chamber 93 will be described with reference to FIGS. 2 to 5.

FIG. 2 is a diagram illustrating an example of a graph indicating the relationship between the concentration of water and internal pressure in a case in which a volatile component is only water. In FIG. 2, the horizontal axis represents the concentration of the target component, such as water, in the measurement chamber 93. The vertical axis represents the pressure in the measurement chamber 93. A graph 901 indicates that when the target component vaporizes in the measurement chamber 93, which is the enclosed space, the internal pressure increases in proportion to the amount of vaporization. Consider the case of measuring the amount of water in the regolith on the actual lunar surface. For example, consider a case in which 0.1 g of the regolith is collected and the amount of water is measured with this method in a concentration range of 0.01 wt% to 10 wt%. In such a case, for example, when the water, i.e. the target component is vaporized in the measurement chamber 93, which is the enclosed space with a temperature of 50°C and an internal volume of 150 $cm^3$, the pressure in the measurement chamber 93 varies in a range of approximately 0.01 kPa to 10 kPa.

FIG. 3 is a diagram illustrating an example of a graph indicating variations of a spectrum of absorbance for water vapor with pressure. FIG. 4 is a diagram illustrating a portion of the graph in FIG. 3 with enlargement. In FIGS. 3 and 4, the horizontal axis represents the wavelength of the laser light applied from the light source 91. The vertical axis represents the absorbance of optical absorption by the target component in the measurement chamber 93. A graph 911 indicates a spectrum of absorbance for water vapor at 0.001 kPa. A graph 912 indicates a spectrum of absorbance for water vapor at 0.01 kPa. A graph 913 indicates a spectrum of absorbance for water vapor at 0.1 kPa. A graph 914 indicates a spectrum of absorbance for water vapor at 1 kPa. A graph 915 indicates a spectrum of absorbance for water vapor at 10 kPa. In general, the spectrum for the target component, such as water vapor, in the near-infrared region to the infrared region is generated by the interaction of light and molecules based on absorption of molecular vibration. As indicated by the graphs 911 to 915, the shape (line shape) of the spectrum is represented by a Voigt function, which is a convolution of a Gaussian distribution function, i.e. the spread due to the Doppler effect, and a Lorentz distribution function, i.e. the spread due to molecular collisions. The graphs 911 to 915 indicate the spectra for water vapor at a given wavelength, at 100% concentration and in a pressure range from 0.001 kPa to 10 kPa at intervals of one digit. The graphs 911 to 915 are based on data from the HITRAN database (https://hitran.org/).

The spectrum of the absorbance non-similarly varies its shape with respect to pressure. As a result, the peak height of the spectrum varies nonlinearly with respect to the amount of water in the regolith. FIG. 5 is a diagram illustrating an example of a graph indicating the relationship between the height of the spectrum of the absorbance and the amount of water. In FIG. 5, the horizontal axis represents the amount of the target component in the measurement chamber 93. Here, the amount of the target component is proportional to the concentration of the target component. The vertical axis represents the peak height of the spectrum. In FIG. 5, a graph 921 has an upward convex curved shape. Thus, the height of the spectrum varies nonlinearly with the amount of water in the regolith. Therefore, if the amount of the target component is measured as a value proportional to the height of the spectrum, a measurement value of the amount contains an error. In other words, in order to measure the amount of the target component with high accuracy based on the height of the spectrum, i.e., a measurement value of the absorbance for the laser light with the certain single wavelength, a pressure correction with a complicated correction curve is required.

As described above, when the content of the heated and vaporized target component is measured over a wide range based on the peak height of the spectrum of the absorbance, the pressure in the enclosed space increases due to the gas component in the enclosed space, and the pressure varies over a wide range depending on the amount of the target component. As a result, there is room for improvement in the measurement accuracy of the measurement apparatus 9 according to the comparative example.

<Embodiment>

An embodiment of the present disclosure will be described below with reference to the drawings. In each drawing, parts having the same configuration or function are denoted with the same reference numeral. In the description of this embodiment, duplicate descriptions of the same parts may be omitted or simplified as appropriate.

The present disclosure aims at measuring the individual content of the target component with high accuracy, using spectral data on the absorbance for the measurement gas, even under a wide range of pressure conditions caused by the target component volatilized by heating and raising the temperature of the sample in the enclosed space. The present disclosure further aims at acquiring relational data between the temperature and the amount of the target component by slowly increasing heating temperature at a predetermined rate using this accurate measurement ability under wide concentration and pressure ranges. This relational data has a shape corresponding to a binding state of the target component to the sample and therefore can be used to analyze a state of existence of the target component in the sample.

FIG. 6 is a diagram illustrating an example of a configuration of a measurement apparatus 1 according to the embodiment. The measurement apparatus 1 measures the amount of a target component, such as water, contained in a sample S, such as regolith, based on tunable diode laser absorption spectroscopy (TDLAS). An example of a case in which the measurement apparatus 1 measures the amount of water (including ice) contained in the sandy regolith in an ultra-high vacuum atmosphere environment, such as the lunar surface, will be described. In such an environment, ultra-high vacuum enclosed space without gas components can be created by shutting off the sample S from the outside after introducing the sample S into the enclosed space.

As illustrated in FIG. 6, the measurement apparatus 1 is provided with an inlet 11, a vaporization chamber 21, a conduit 22, a measurement chamber 23, a light emitter 33, a light receiver 52, and an arithmetic unit 60. The measurement apparatus 1 is provided with a window 41 between the light emitter 33 and the measurement chamber 23. The measurement apparatus 1 is provided with a window 42 between the measurement chamber 23 and the light receiver 52.

The inlet 11 is an entrance to introduce the sample S, such as the regolith, into the inside of the measurement apparatus 1. The inlet 11 is spatially connected to the vaporization chamber 21. A user can introduce the sample S into the vaporization chamber 21 from the inlet 11. The inlet 11 is provided with a valve 12 to shut off space from the outside. After introduction of the sample S, the valve 12 can be closed to seal a series of spaces constituted of the vaporization chamber 21, the conduit 22, and the measurement chamber 23.

In the vaporization chamber 21, the sample S is placed. The vaporization chamber 21 is provided with a heater 24. The heater 24 vaporizes the target component contained in the sample S by heating the sample S and controlling the temperature of the sample S.

The conduit 22 spatially connects the vaporization chamber 21 and the measurement chamber 23. The target component vaporized in the vaporization chamber 21 spreads via the conduit 22 through the enclosed space constituted of the vaporization chamber 21, the conduit 22, and the measurement chamber 23.

The measurement chamber 23 is a space in which laser light R is applied to the vaporized target component, in order to measure the absorbance.

The light emitter 33 outputs the laser light R to the measurement chamber 23. The light emitter 33 is provided with a light source 31 and an optical system 32. The light source 31 is a source of the laser light R. The light source 31 may be, for example, a wavelength tunable semiconductor laser capable of varying the wavelength of the laser light R. The optical system 32 includes optical elements such as lenses.

The laser light R output from the light emitter 33 passes through the measurement chamber 23 via the window 41 and is partially absorbed by the target component. The laser light R that has passed through the measurement chamber 23 reaches the light receiver 52 via the window 42.

The light receiver 52 is provided with a detector 51. Upon detecting the laser light R, the detector 51 outputs, to the arithmetic unit 60, an electrical signal corresponding to the light intensity thereof, as a light-receiving signal. The detector 51 may be, for example, a photodiode having a photoelectric conversion effect. The detector 51 is electrically connected to the arithmetic unit 60.

The arithmetic unit 60 analyzes the light-receiving signal received from the light receiver 52 to calculate the amount of the target component contained in the sample S. The arithmetic unit 60 is provided with a controller 61, a memory 62, and an input/output interface 63. The controller 61 has one or more processors and controls operations of the arithmetic unit 60. The memory 62 stores data including measurement data, and programs. The memory 62 may include, for example, random access memory (RAM), read only memory (ROM), and storage (e.g., a solid state drive (SSD), a magnetic hard disk, or the like). The input/output interface 63 accepts operation input from the user and displays the arithmetic result of the arithmetic unit 60 to the user. The input/output interface 63 may include an input device and a display device. For example, the input/output interface 63 may include, as the input device, a keyboard, a pointing device, a touch panel, or the like. The input/output interface 63 may include, as the output device, a liquid crystal panel, an organic electro-luminescence (EL) display, or the like.

In the above configuration, the measurement apparatus 1 acquires the amount of the target component based on an integral value of the absorbance obtained from the intensity of the laser light R absorbed by the target component while the laser light R passes through the interior of the measurement chamber 23. Specifically, the measurement apparatus 1 acquires, by analyzing the light-receiving signal, a spectrum of the absorbance of the laser light R absorbed by the target component while the laser light R passes through the interior of the measurement chamber 23, and acquires the amount of the target component based on the integral value of the spectrum of the absorbance. Therefore, according to the measurement apparatus 1, the amount of the target component can be measured with higher accuracy even when pressure in the measurement chamber 23 significantly varies due to vaporization of the target component in the enclosed space.

FIG. 7 is a flowchart illustrating an example of operations of the measurement apparatus 1 in FIG. 6. The operations of the measurement apparatus 1 described with reference to FIG. 7 may correspond to one of measurement methods of the measurement apparatus 1. As a precondition for the following operations, the measurement apparatus 1 is installed at a low temperature at the level of not vaporizing ice, in an ultra-high vacuum state (ultra-low pressure state) similar to the lunar surface. In an initial state, the valve 12 of the measurement apparatus 1 is open. The heater 24 is in an OFF state. Since the vaporization chamber 21 is open to the outside and the heater is in the OFF state, the vaporization chamber 21 is at the low temperature at the level of not vaporizing ice, in the ultra-high vacuum state (ultra-low pressure state) similar to the lunar surface. The following operations are started before the sample S is introduced into the vaporization chamber 21.

In step S1, the measurement apparatus 1 starts outputting the laser light R from the light source 31 and receiving the laser light R at the detector 51. In this embodiment, the measurement apparatus 1 operates even before the sample S is introduced into the vaporization chamber 21, and starts measuring the spectrum of the absorbance for water, which is the target component.

In step S2, the measurement apparatus 1 accepts introduction of the sample S by the user. Specifically, the user introduces the sample S, such as the regolith, into the vaporization chamber 21 from the inlet 11, and closes the valve 12 to enclose the space constituted of the vaporization chamber 21, the conduit 22, and the measurement chamber 23.

In step S3, the measurement apparatus 1 heats the sample S with the heater 24 to control the temperature of the sample S to a desired value. This vaporizes the water, which is the target component contained in the sample S, as well as ice. This water diffuses into the measurement chamber 23 through the conduit 22. Since the vaporization chamber 21, the conduit 22, and the measurement chamber 23 constitute the enclosed space, the concentration of the target component in the measurement chamber 23 increases within a range not exceeding the saturated vapor pressure, as the target component vaporizes. In this process, the measurement apparatus 1 continues to measure the spectrum of the absorbance for the target component by the light emitter 33 and the light receiver 52.

In step S4, the measurement apparatus 1 acquires the light-receiving signal of the laser light R that has passed through the space containing the target component in the measurement chamber 23. Specifically, the detector 51 outputs, to the arithmetic unit 60, a signal indicating the intensity of the detected laser light R, as the light-receiving signal.

In step S5, the measurement apparatus 1 acquires the amount of the target component based on a value of the area (integral value) of the spectrum of the absorbance calculated from the intensity of the laser light R absorbed by the target component. Details of the process of acquiring the amount of the target component will be described below.

In step S6, the measurement apparatus 1 outputs the amount of the target component acquired in step S5. Specifically, the measurement apparatus 1 stores a measurement value of the amount of the target component in the memory 62 or displays the measurement value on the input/output interface 63. After completing the process of step S6, the measurement apparatus 1 ends the processes of the flowchart. After these operations, the measurement apparatus 1 stops heating by the heater 24 and cools down. The user opens the valve 12 to discharge the sample S and to open the enclosed space inside the measurement apparatus 1 to the outside. As a result, the vaporization chamber 21 and the measurement chamber 23 quickly return to the ultra-high vacuum state. When there is another sample S to be measured, the user can measure a target component contained in that sample S.

Next, the details of the process of acquiring the amount of the target component in step S5 will be described with reference to FIGS. 8 to 13.

FIG. 8 is a diagram schematically illustrating variation of laser drive current with time. In FIG. 8, the horizontal axis indicates time. The vertical axis indicates the magnitude of the laser drive current that drives the light source 31. A graph 781 indicate the variation of the laser drive current with time. In the example in FIG. 8, the graph 781 indicates that the laser drive current fluctuates between $i_0$ and $i_2$ with a period $t_0$.

FIG. 9 is a diagram schematically illustrating a graph indicating the relationship between the laser drive current and wavelength. In FIG. 9, the horizontal axis indicates the magnitude of the laser drive current that drives the light source 31. The vertical axis indicates the wavelength of the laser light R to be output from the light source 31. A graph 801 indicates the relationship between the laser drive current and the wavelength when the temperature T of the light source 31 is 20°C. A graph 802 indicates the relationship between the laser drive current and the wavelength when the temperature T of the light source 31 is 25°C. A graph 803 indicates the relationship between the laser drive current and the wavelength when the temperature T of the light source 31 is 30°C. As illustrated in the graphs 801 to 803, at a constant temperature, the magnitude of the laser drive current and the wavelength have a linear relationship with each other. At a constant laser drive current, the wavelength of the laser light R tends to increase as the temperature increases. Therefore, the measurement apparatus 1 controls, in advance, the wavelength of the laser light R to be output from the light source 31 as coinciding with an absorption line center wavelength $\lambda_1$ at a specific wavelength that water vapor, i.e. the target component has. Specifically, as illustrated in FIG. 9, the oscillation wavelength of the semiconductor laser shifts with temperature. Therefore, the measurement apparatus 1 controls the temperature of the light source 31 to a temperature corresponding to the absorption line center wavelength $\lambda_1$. In the example in FIG. 9, when the laser drive current fluctuates between $i_0$ and $i_2$, the wavelength of the laser light R to be output from the light source 31 fluctuates, at the temperature T = 25°C, in a range from $\lambda_0$ to $\lambda_2$ including the absorption line center wavelength $\lambda_1$ of the target component.

As illustrated in the graph 781 in FIG. 8, in which the laser drive current varies periodically with the period $t_0$, the magnitude of the laser drive current has a linear relationship with the wavelength of the laser light R, as illustrated in FIG. 9. Therefore, when the laser drive current varies as illustrated in the graph 781 in FIG. 8, the wavelength of the laser light R also varies periodically. In other words, the light source 31 outputs the laser light R with wavelength swept in the range including the absorption line center wavelength $\lambda_1$ of the light to be absorbed by the target component.

FIG. 10 is a diagram schematically illustrating variation, with time, of the intensity of the light-receiving signal detected by the detector 51. In FIG. 10, the horizontal axis indicates time. The vertical axis indicates the intensity of the light-receiving signal. In FIG. 10, a graph 782 indicates that the intensity of the light-receiving signal fluctuates in a range from $I_0$ to $I_2$ with the period $t_0$. As comparing the shape of the graph 782 with that of the graph 781 in FIG. 8, the intensity of the light-receiving signal does not increase linearly in the graph 782 and has a depression 783 in part. The depression 783 indicates that the laser light R is absorbed by the target component at the absorption wavelength of the target component while the laser light R passes through the interior of the measurement chamber 23. As described below, in this embodiment, the measurement apparatus 1 converts the intensity of the light-receiving signal into the absorbance, and calculates the area (integral value) of the spectrum of the absorbance to acquire the amount of the target component.

The calculation of the area of a portion corresponding to the depression 783 will be described with reference to FIGS. 11 to 13. FIG. 11 is a diagram schematically illustrating the relationship between laser wavelength and the intensity of the light-receiving signal. In FIG. 11, the horizontal axis indicates the wavelength of the laser light R. The vertical axis indicates the intensity of the light-receiving signal detected by the detector 51. A graph 811 indicates the relationship between the wavelength of the laser light R and the intensity of the light-receiving signal at a certain period of the periodically varying laser light R. As described above, the wavelength of the laser light R is proportional to the laser drive current (FIG. 9), and at a certain period, the laser drive current has a linear relationship with time as illustrated in FIG. 8. Therefore, the wavelength in FIG. 11 is a value corresponding to time with a linear relationship at a certain period of swept wavelength. In the example of FIG. 11, a graph 811 has a depression around the absorption line center wavelength λ1. The area of the depression corresponds to the area of the depression 783 in FIG. 10. Therefore, the measurement apparatus 1 calculates the area of a portion corresponding to a region 813, by determining the region 813 enclosed by an asymptote 812 of the graph 811 and the graph 811 and converting the region 813 into the absorbance. Specifically, the measurement apparatus 1 first subjects the light-receiving signal to baseline correction, in the wavelength range from $\lambda_0$ to λ1, by calculating the difference in the intensity of the light-receiving signal between the asymptote 812 and the graph 811 for each wavelength.

FIG. 12 is a diagram schematically illustrating the relationship between the laser wavelength and the intensity of the light-receiving signal processed by the baseline correction. In FIG. 12, the horizontal axis indicates the wavelength of the laser light R. The vertical axis indicates the intensity of the light-receiving signal detected by the detector 51. A graph 821 illustrates a curve in which the graph 811 in FIG. 11 is processed by the baseline correction. An asymptote (baseline signal) 822 indicates a curve in which the asymptote 812 in FIG. 11 is processed by the baseline correction. A region 823 corresponds to the region 813 in FIG. 11.

FIG. 13 is a diagram schematically illustrating the relationship between the laser wavelength and the absorbance. In FIG. 13, the horizontal axis indicates the wavelength of the laser light R. The vertical axis indicates the absorbance of the laser light R. A graph 831 is acquired by converting the graph 821 in FIG. 12 into the absorbance. That is, the measurement apparatus 1 acquires the absorbance $H = \ln(I_0/I_t)$ by calculating the ratio between the intensity $I_0$ of the laser light R output from the light source 31 and the intensity It of the laser light R passing through the measurement chamber 23 and detected by the detector 51.

The graph 831 has a shape indicated by the Voigt function described above. It is known that the area A of a region 841 enclosed by the graph 831 and the horizontal axis is proportional to the concentration C of the target component, the optical path length L of the laser, and pressure P. Here, the area A corresponds to a value obtained by integrating the spectrum of the absorbance indicated by the graph 831 by wavelength. The pressure P is a pressure in the enclosed space due to generation of all volatile gases including the target component. In other words, when k represents a proportionality coefficient, the concentration C of the target component is calculated by the following equation 2 based on the area A, the optical path length L of the laser, and the pressure P.

(Equation 2)

$$C = k \times A/(L \times P)$$

When V represents the volume of the enclosed space constituted of the vaporization chamber 21, the conduit 22, and the measurement chamber 23, the molar mass M of the target component in the enclosed space is calculated by the following equation 3.

(Equation 3)

$$M = C \times P \times V = (k \times A \times V)/L$$

Therefore, for the target component with a known mass M, the proportionality coefficient k can be calculated in advance by calculating the area A under the conditions of a known laser optical path length L and volume V. The measurement apparatus 1 stores such proportionality coefficient k, volume V, and optical path length L in the memory 62 in advance. Upon calculating the area A of the spectrum of the absorbance of the laser light R, the measurement apparatus 1 can acquire, based on the equation 3, the total amount (mass) of the target component volatilized from the sample S.

FIG. 14 is a diagram that calculates, in the enclosed space, the relationship between the height and area of the spectrum of the absorbance for water (gas) at a certain wavelength and the amount of the target component (in this example, water), using the HITRAN database. In FIG. 14, the horizontal axis indicates the amount of the target component. The vertical axis indicates the height or area of the spectrum of the absorbance. A graph 851 indicates the relationship between the area of the spectrum of the absorbance and the amount of the target component. A graph 921 indicates the relationship between the peak height of the spectrum of the absorbance and the amount of the target component. In FIG. 14, the pressure increases with increase in the amount of the target component due to the enclosed space, and in this example, the graphs 851 and 921 for a range of pressure from 0.001 k to 10 kPa are illustrated. As indicated by the graph 921, the peak height of the spectrum varies nonlinearly with the amount of the target component at a high pressure range. In contrast, as indicated by the graph 851, the area (integral value) of the spectrum varies linearly with the amount of the target component over the entire pressure range. Therefore, the measurement apparatus 1 can measure the amount of the target component in the sample S with high accuracy, based on the area (integral value) of the spectrum using the equation 3 over the wide ranges of pressure and the amount of the target component.

In the case of measuring water, as the target component, the value of the area (integral value) of the spectrum of the absorbance for water vapor maintains linearity with respect to the amount of the target component (amount of water) even when the main component of volatile components contained in the sample S is not water, that is, when the sample S contains more other components than water. In other words, even when the target component is a substance other than water and volatile components other than the target component are present in the enclosed space, it is known that the amount of the target component and the area of the spectrum of the absorbance have a linear relationship over a wide pressure range, regardless of the amounts of substances other than the target component. Therefore, even when the sample S contains components other than the target component of interest, the measurement apparatus 1 can measure the amount of the target component with high accuracy.

The measurement apparatus 1 may calculate the area of the spectrum of the absorbance (e.g., the area of the region 841 in FIG. 13) by integrating the graph 831 in FIG. 13 with respect to wavelength, or may calculate the area by other methods. For example, focusing on that the spectrum of the absorbance has the shape of the Voigt function, the measurement apparatus 1 may perform curve fitting of the graph 831 to a model equation of the Voigt function or the like, and calculate the area using data fitted to the complete Voigt function. The measurement apparatus 1 may also calculate the area of the spectrum by limiting part of the region centered on the peak wavelength of the spectrum, rather than the entire wavelength of spectrum formation, within an error allowable extent. By performing these processes, the measurement apparatus 1 can calculate the amount of the target component with higher accuracy by measuring the spectrum for the target component with higher accuracy. The measurement apparatus 1 may also calculate the area (integral value) of part of the region of the spectrum centered on the peak wavelength of the spectrum, rather than the entire wavelength range of the spectrum of the absorbance, within an error allowable extent.

The measurement apparatus 1 can be used not only to calculate the amount of the target component contained in the sample S, but also to determine the binding state of the target component contained in the sample S. For example, water can exist in the sample S as free water, bound water, surface-attached water, internal water, or the like. These types of water are identical as molecules, but vaporize at different temperatures depending on the binding state. Therefore, the relationship between the temperature of the sample S and the amount of water molecules that have vaporized from the sample S and come to exist in the enclosed space indicates the distribution of the binding state of water in the sample S. In other words, the relational data between the temperature and the amount of volatilization makes it possible to analyze the binding state of the original solid sample S and the volatile component of the target component. Therefore, the measurement apparatus 1 may measure the amount of the target component, which is the vaporized contained volatile component, while heating the sample S in the enclosed space with the heater 24 and slowly increasing the temperature of the sample S at a constant rising rate. The measurement apparatus 1 may output relational data that indicates the correspondence between the temperature of the sample S and the amount of the target component calculated by analyzing the light-receiving signal of the laser light R.

FIG. 15 is a diagram illustrating an example of the relational data indicating the correspondence between the temperature and the amount of the target component. In FIG. 15, the horizontal axis indicates temperature. The vertical axis indicates the amount of the target component. A graph 861 is the relational data indicating the correspondence between the temperature and the amount of the target component. The user can analyze the binding state between the original solid sample S and the volatilized target component based on temperatures at which the amount of the target component rapidly increases, the amounts of the increase, and the like.

The measurement apparatus 1 can also measure, for the target components other than water, the amounts of the target components attached to the sample S with high accuracy by applying light with wavelengths to be absorbed by the target components. For example, the measurement apparatus 1 may measure, as the target components, carbon monoxide, carbon dioxide, oxygen, ammonia, and other components contained in the sample S.

As described above, the measurement apparatus 1 is provided with the vaporization chamber 21, the measurement chamber 23, the light source 31, the detector 51, and the arithmetic unit 60. In the vaporization chamber 21, the sample S containing the target component is placed. The measurement chamber 23 is spatially connected to the vaporization chamber 21 and forms the enclosed space integrally with the vaporization chamber 21. The light source 31 applies, to the measurement chamber 23, the laser light R whose wavelength varies. The detector 51 detects the laser light R that has been applied from the light source 31 and has passed through the measurement chamber 23. The arithmetic unit 60 calculates the amount of the target component contained in the sample S by analyzing the light-receiving signal of the laser light R detected by the detector 51. Here, the arithmetic unit 60 acquires the amount of the target component based on the value of the area (integral value) of the spectrum of the absorbance calculated from the laser light R that is absorbed by the target component while the laser light R passes through the interior of the measurement chamber 23. Therefore, according to the measurement apparatus 1, the amount of the target component can be measured with high accuracy even when the pressure in the enclosed space fluctuates significantly.

The arithmetic unit 60 may also acquire the curve data by performing the curve fitting of the spectrum of the absorbance of the laser light R to the model equation, e.g. the Voigt function, and acquire the area of the spectrum based on the curve data.

Thus, the measurement apparatus 1 acquires the area of the spectrum by performing the curve fitting of the spectrum of the absorbance of the laser light R, thus reducing calculation cost and outputting the amount of the target component at high speed.

The light source 31 may be the wavelength tunable semiconductor laser capable of varying the wavelength of the laser light R to be output. Thus, using the wavelength tunable semiconductor laser, as the light source 31, allows the measurement apparatus 1 to measure the amount of the target component based on the area of the spectrum by the wavelength sweep in the range including the absorption line center wavelength $\lambda_1$ of the target component.

The light source 31 may apply, as the laser light R, the light whose wavelength varies periodically. The arithmetic unit 60 may acquire the spectrum for each of the plurality of periods with which the wavelength of the laser light R varies, and acquire the amount of the target component based on the plurality of spectra. The measurement apparatus 1 acquires the spectrum for each of the plurality of periods with which the wavelength of the laser light R varies and acquires the amount of the target component based on the plurality of spectra, thus allowing the measurement of the amount of the target component with even higher accuracy with reduced influence of variations in data from period to period.

The measurement apparatus 1 may further be provided with the heater 24 that controls the temperature of the sample S. The arithmetic unit 60 may output the relational data indicating the correspondence between the temperature of the sample S and the amount of the target component calculated by analyzing the light-receiving signal of the laser light R. The measurement apparatus 1 outputs the relational data indicating the correspondence between the temperature of the sample S and the amount of the target component, thus allowing the analysis of the binding state of the target component in the sample S based on the relational data.

The present disclosure is not limited to the embodiment described above. For example, a plurality of blocks described in the block diagram may be integrated, or one block may be divided. A plurality of steps described in the flowchart may be executed in parallel or in a different order, instead of being executed in chronological order according to the description, depending on the processing capability of the apparatus performing each step or as needed. Other variations may be possible to the extent of not departing from the intent of the present disclosure.

## Claims

**1.** A measurement apparatus comprising:

a vaporization chamber in which a sample containing a target component is placed;
a measurement chamber spatially connected to the vaporization chamber, the measurement chamber configured to form enclosed space integrally with the vaporization chamber;
a light source configured to apply, to the measurement chamber, laser light whose wavelength varies;
a detector configured to detect the laser light that has been applied from the light source and has passed through the measurement chamber; and
an arithmetic unit configured to calculate an amount of the target component contained in the sample by analyzing a light-receiving signal of the laser light detected by the detector,
wherein the arithmetic unit is configured to acquire the amount of the target component, based on an integral value of a spectrum of absorbance of the laser light that is absorbed by the target component while the laser light passes through an interior of the measurement chamber.

**2.** The measurement apparatus according to claim 1, wherein the arithmetic unit is configured to acquire curve data by performing curve fitting of the spectrum of the absorbance of the laser light to a model equation, and acquire the integral value of the spectrum based on the curve data.

**3.** The measurement apparatus according to claim 1 or 2, wherein the light source is a wavelength tunable semiconductor laser configured to be able to vary the wavelength of the laser light to be output.

**4.** The measurement apparatus according to any one of claims 1 to 3, wherein

the light source is configured to apply, as the laser light, light whose wavelength varies periodically, and
the arithmetic unit is configured to acquire the spectrum for each of a plurality of periods with which the wavelength of the laser light varies, and acquire the amount of the target component based on a plurality of the spectra.

**5.** The measurement apparatus according to any one of claims 1 to 4, further comprising:

a heater configured to control temperature of the sample,
wherein the arithmetic unit is configured to output relational data indicating a correspondence between the temperature of the sample and the amount of the target component calculated by analyzing the light-receiving signal of the laser light.

**6.** A measurement method by a measurement apparatus, the measurement method comprising:

applying, by a light source to a measurement chamber, laser light whose wavelength varies, the measurement chamber being spatially connected to a vaporization chamber in which a sample containing a target component is placed, the measurement chamber configured to form enclosed space integrally with the vaporization chamber;
detecting, by a detector, the laser light that has been applied from the light source and has passed through the measurement chamber; and
calculating, by an arithmetic unit, an amount of the target component contained in the sample by analyzing a light-receiving signal of the laser light detected by the detector,
wherein the arithmetic unit is configured to acquire the amount of the target component, based on an integral value of a spectrum of absorbance of the laser light that is absorbed by the target component while the laser light passes through an interior of the measurement chamber.

**7.** The measurement method according to claim 6, wherein the arithmetic unit is configured to acquire curve data by performing curve fitting of the spectrum of the absorbance of the laser light to a model equation, and acquire the integral value of the spectrum based on the curve data.

**8.** The measurement method according to claim 6 or 7, wherein the light source is a wavelength tunable semiconductor laser configured to be able to vary the wavelength of the laser light to be output.

**9.** The measurement method according to any one of claims 6 to 8, wherein

the light source is configured to apply, as the laser light, light whose wavelength varies periodically, and

the arithmetic unit is configured to acquire the spectrum for each of a plurality of periods with which the wavelength of the laser light varies, and acquire the amount of the target component based on a plurality of the spectra.

**10.** The measurement method according to any one of claims 6 to 9, further comprising:

controlling, by a heater, temperature of the sample,

wherein the arithmetic unit is configured to output relational data indicating a correspondence between the temperature of the sample and the amount of the target component calculated by analyzing the light-receiving signal of the laser light.

FIG. 1

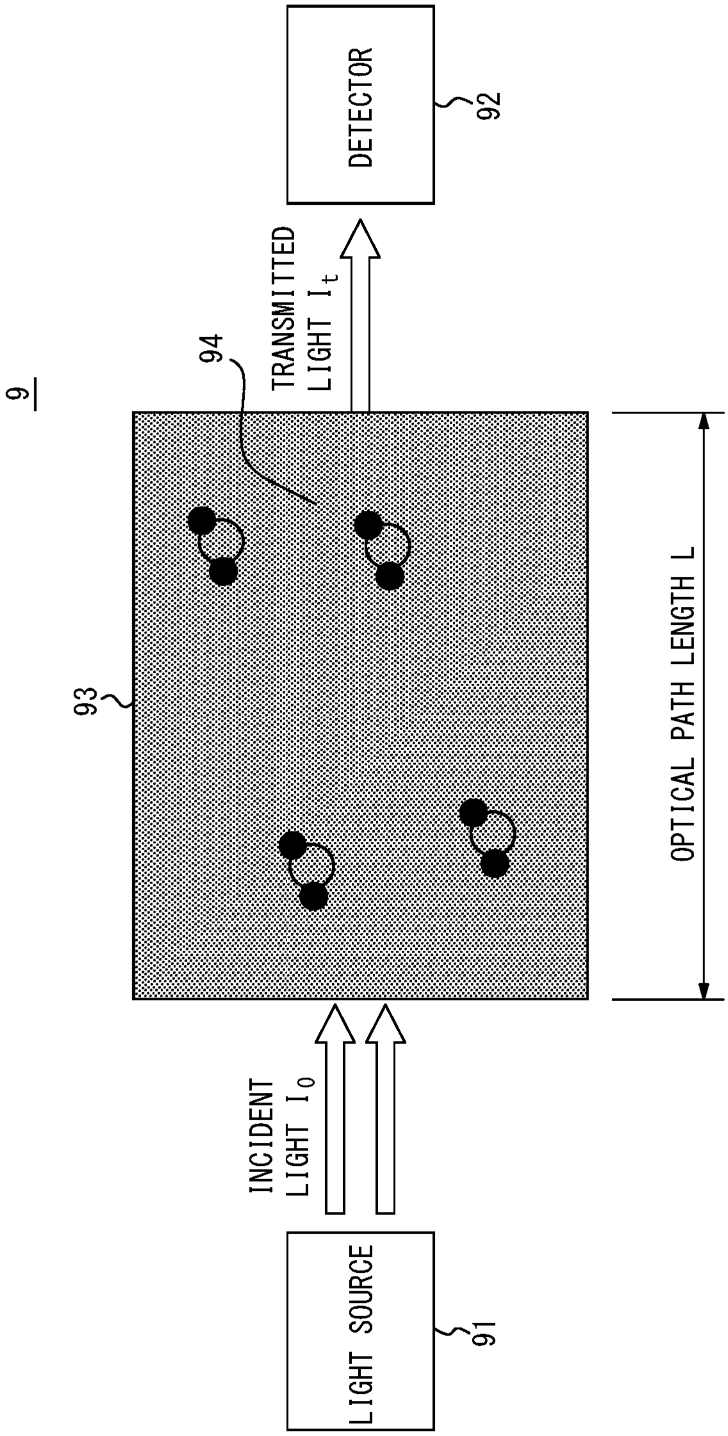
9
LIGHT SOURCE
91
INCIDENT
LIGHT $I_0$
93
94
TRANSMITTED
LIGHT $I_t$
DETECTOR
92
OPTICAL PATH LENGTH L

FIG. 2

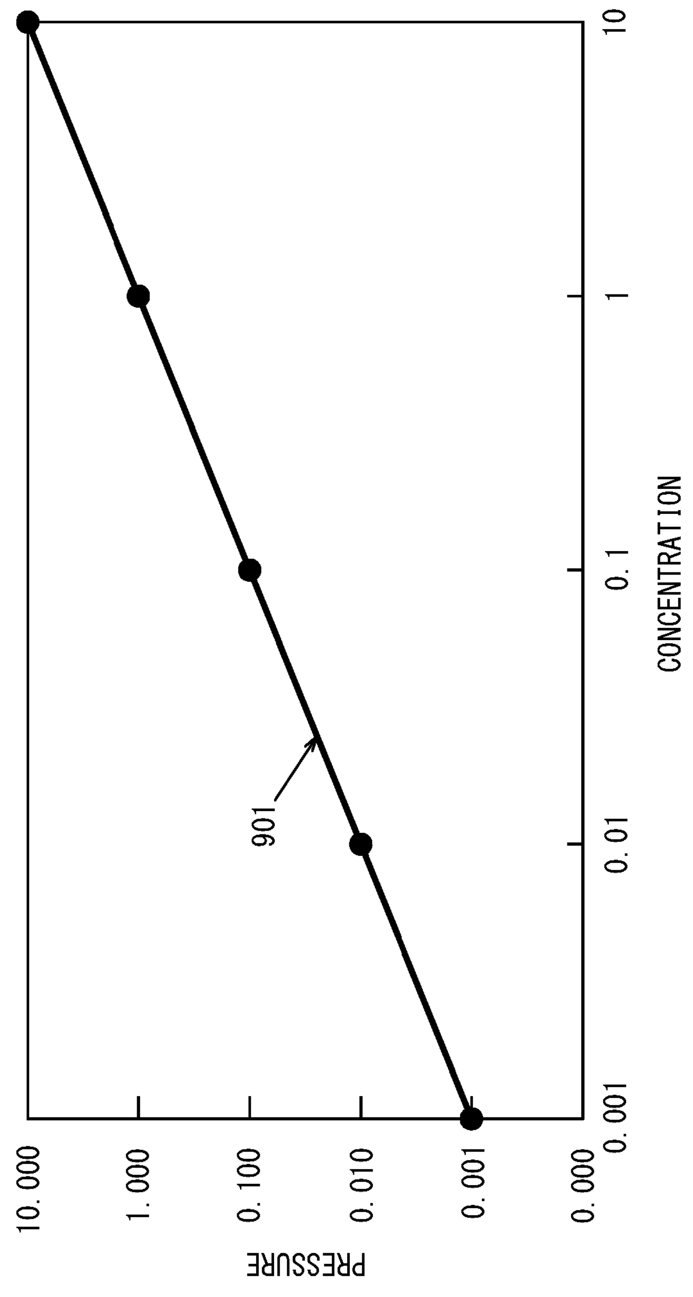
PRESSURE
10.000
1.000
0.100
0.010
0.001
0.000
CONCENTRATION
0.001
0.01
0.1
1
10
901

FIG. 3

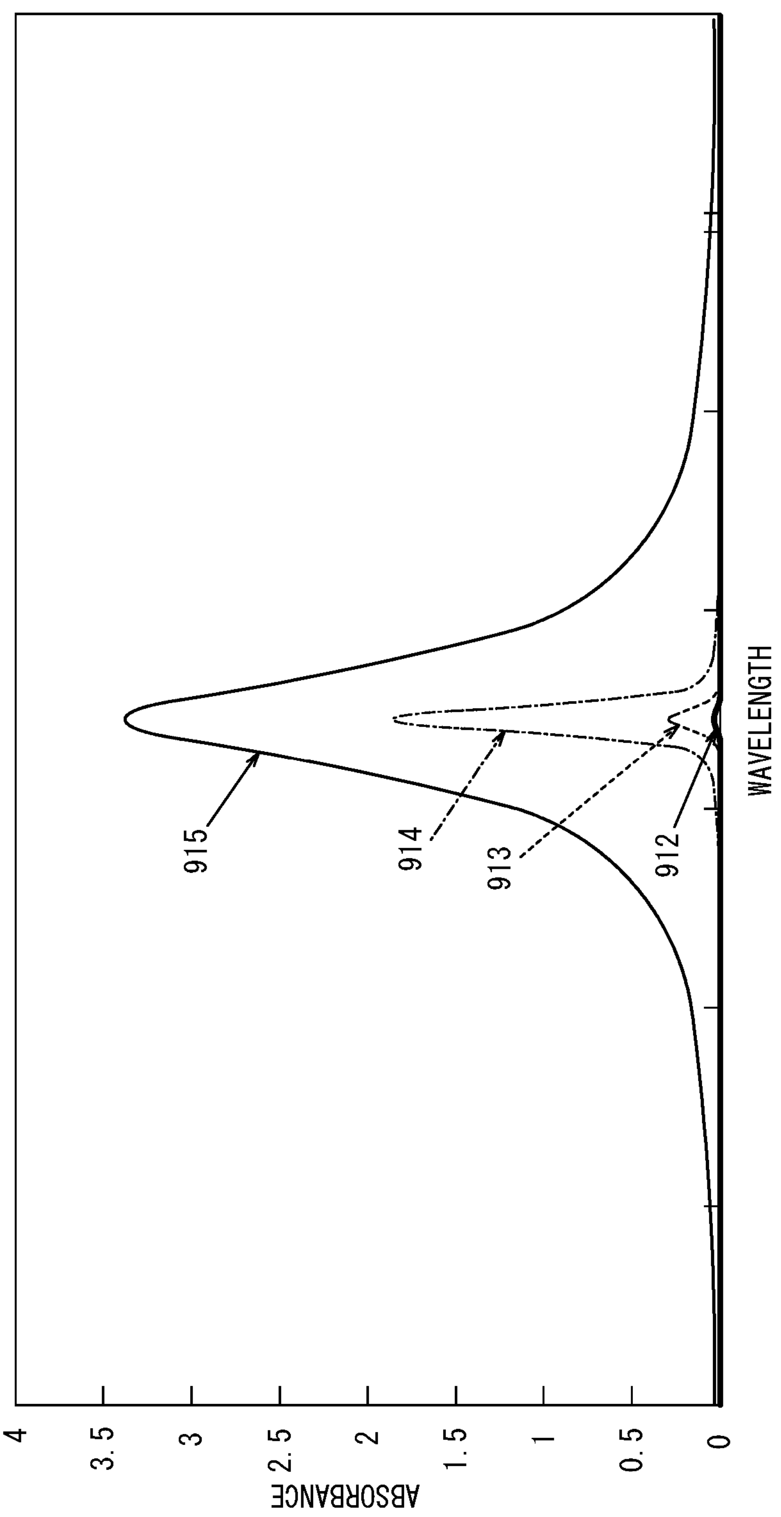

FIG. 4

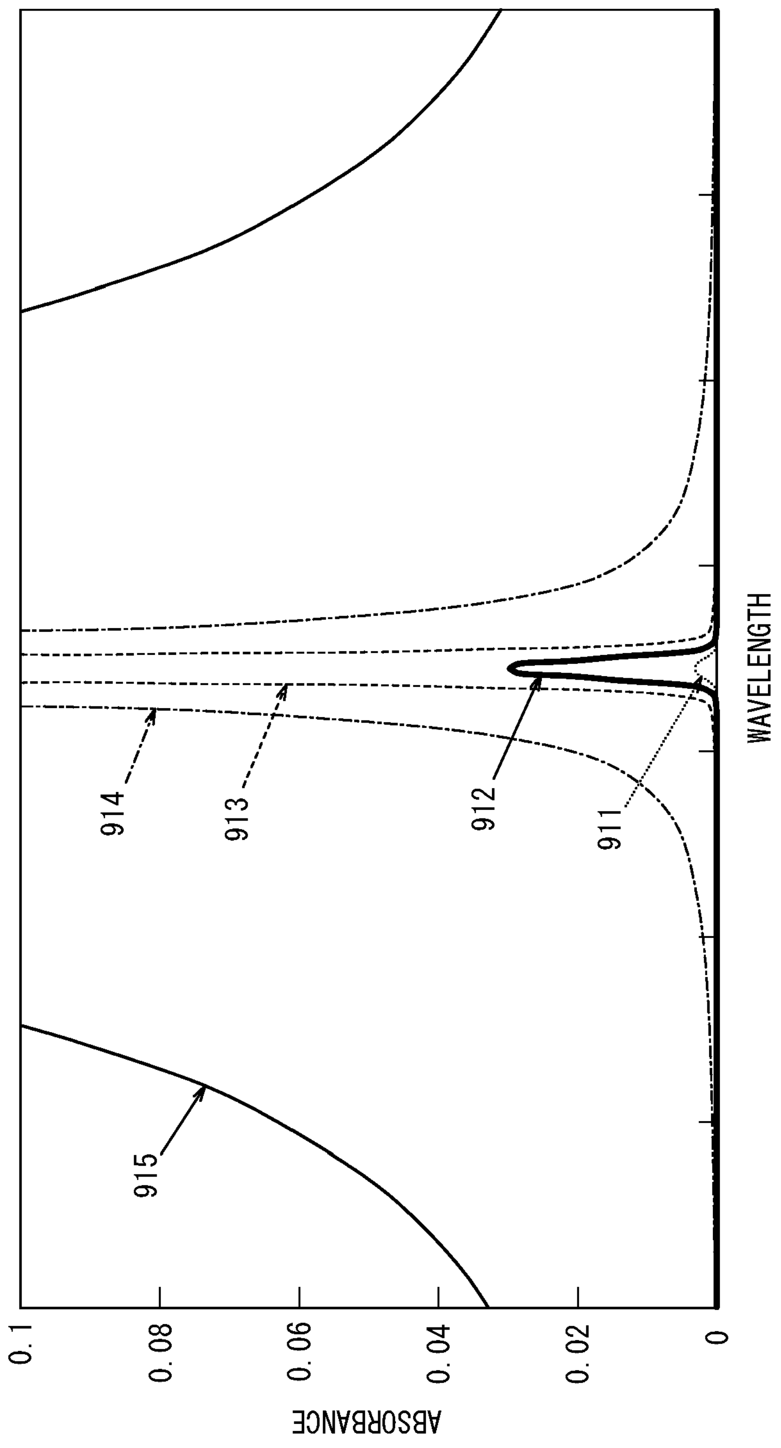
ABSORBANCE
0.1
0.08
0.06
0.04
0.02
0
WAVELENGTH
911
912
913
914
915

FIG. 5

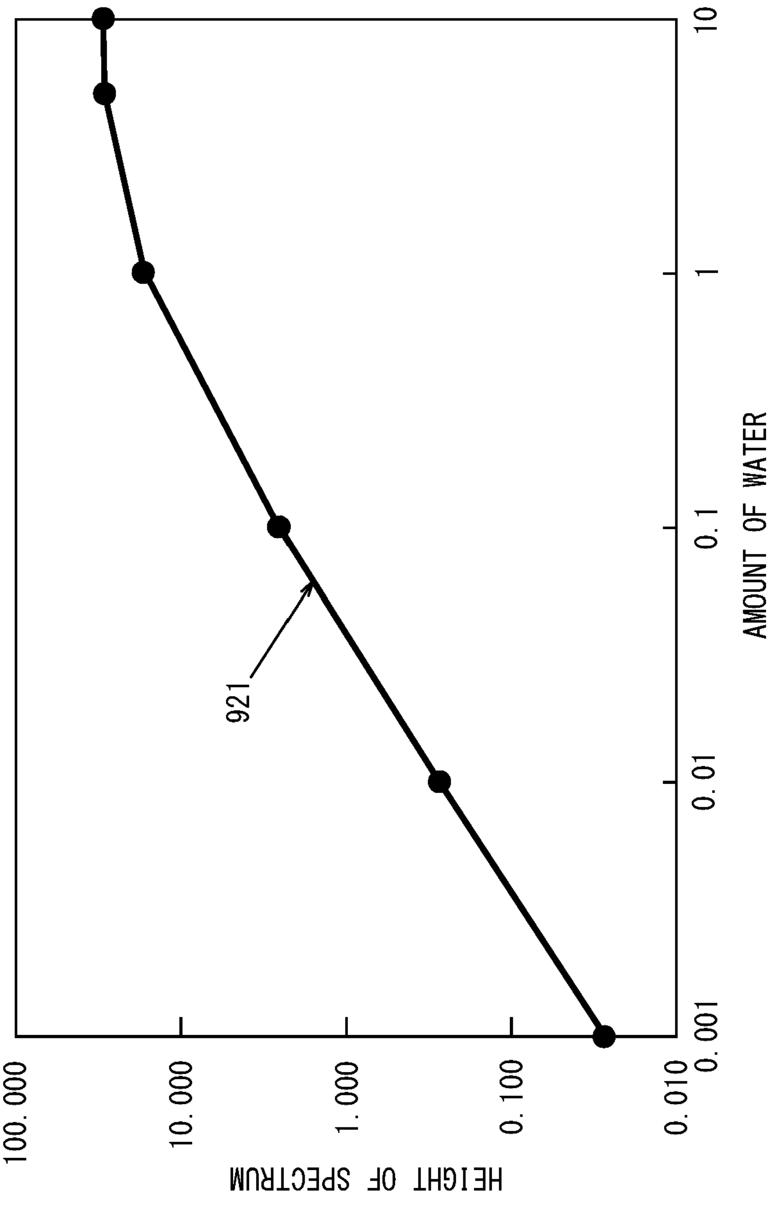
100.000
10.000
1.000
0.100
0.010
HEIGHT OF SPECTRUM
0.001
0.01
0.1
1
10
AMOUNT OF WATER
921

FIG. 6

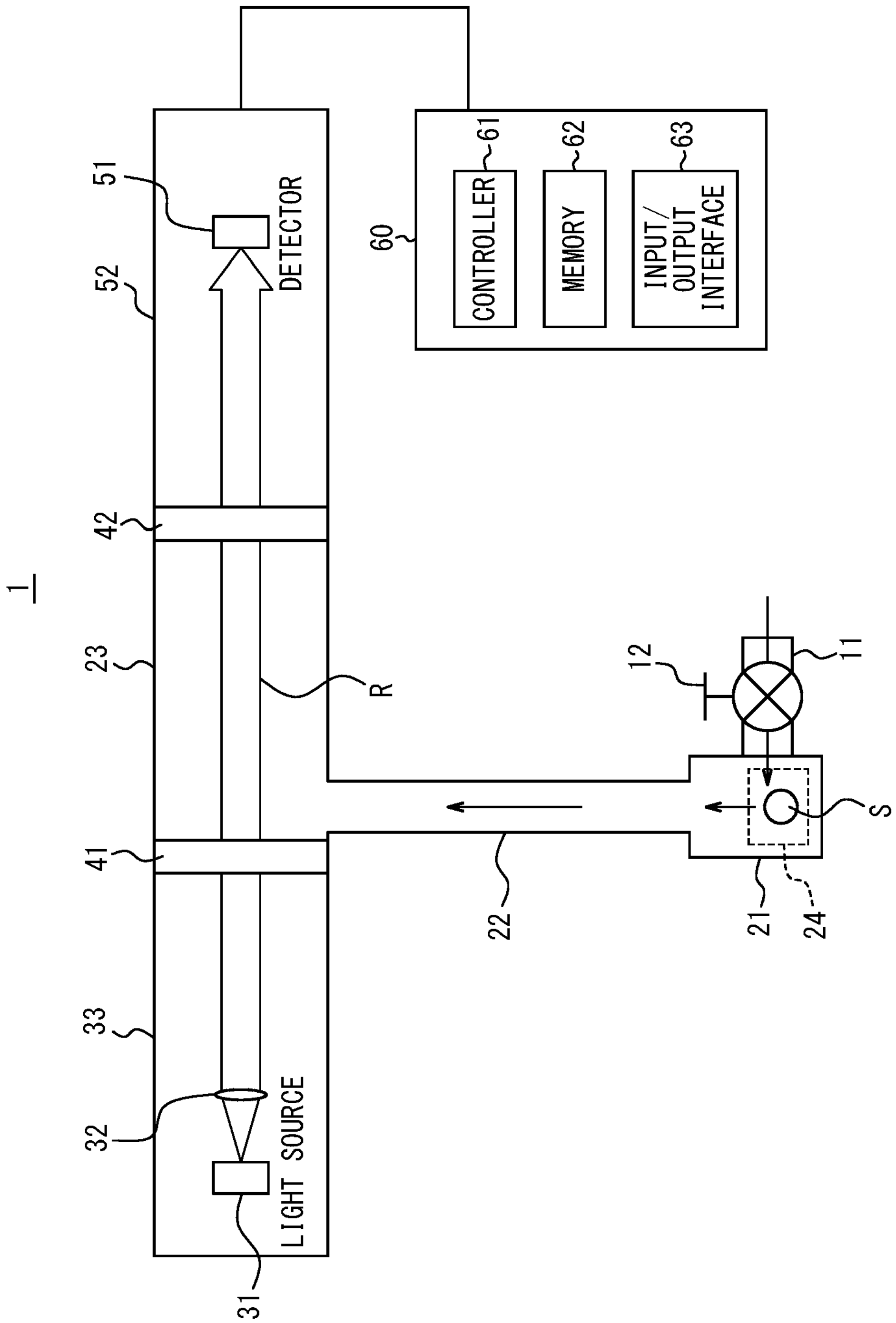
1
51
DETECTOR
52
60
CONTROLLER
61
MEMORY
62
INPUT/
OUTPUT
INTERFACE
63
42
23
R
12
11
41
22
21
24
S
33
32
LIGHT SOURCE
31

FIG. 7

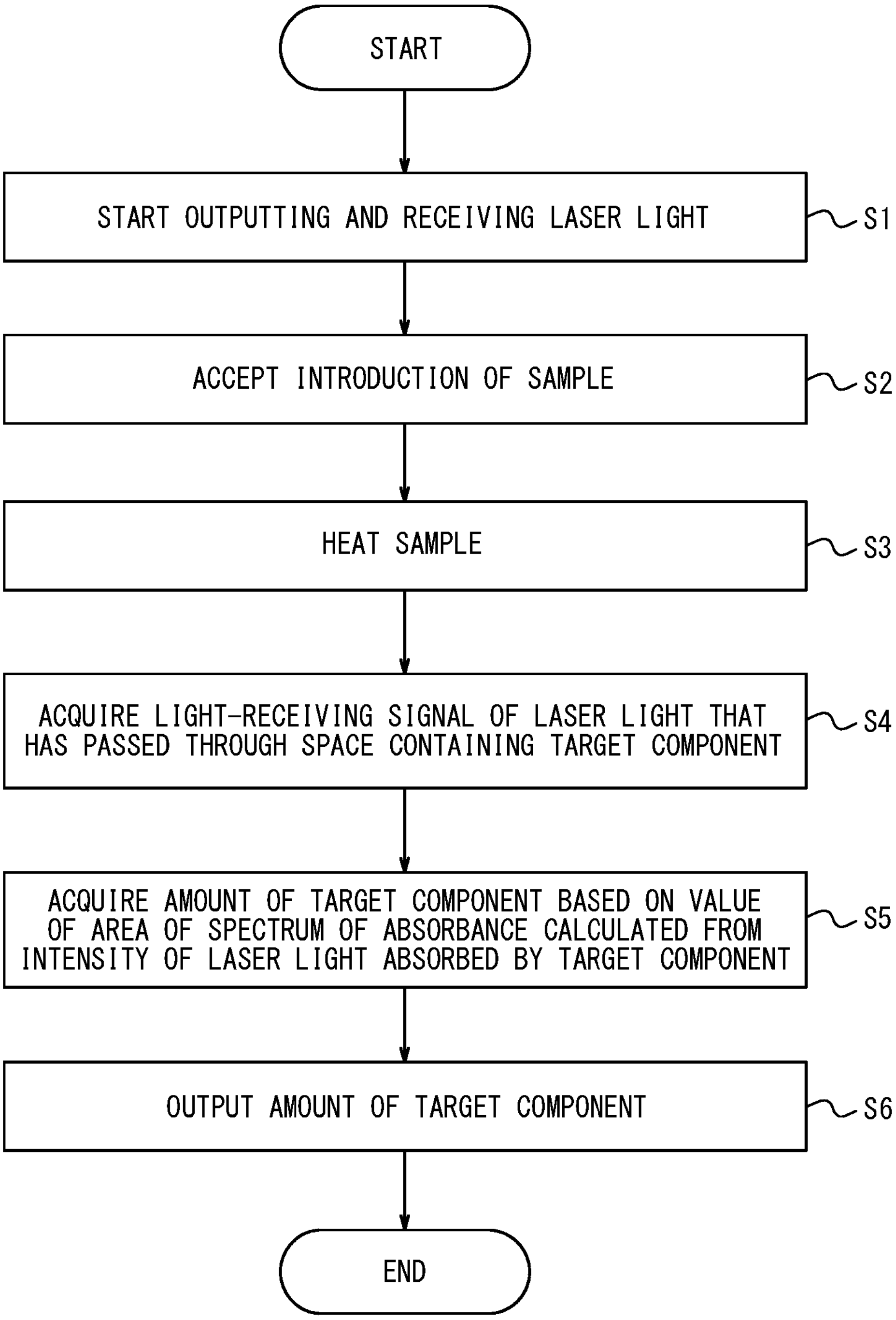
START
START OUTPUTTING AND RECEIVING LASER LIGHT
S1
ACCEPT INTRODUCTION OF SAMPLE
S2
HEAT SAMPLE
S3
ACQUIRE LIGHT-RECEIVING SIGNAL OF LASER LIGHT THAT HAS PASSED THROUGH SPACE CONTAINING TARGET COMPONENT
S4
ACQUIRE AMOUNT OF TARGET COMPONENT BASED ON VALUE OF AREA OF SPECTRUM OF ABSORBANCE CALCULATED FROM INTENSITY OF LASER LIGHT ABSORBED BY TARGET COMPONENT
S5
OUTPUT AMOUNT OF TARGET COMPONENT
S6
END

FIG. 8

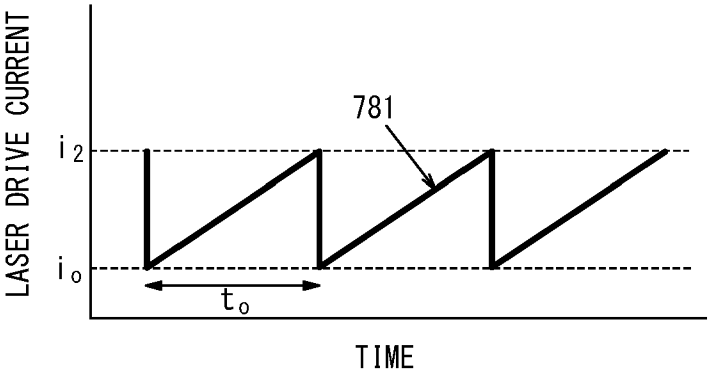
LASER DRIVE CURRENT
781
i 2
i o
t o
TIME

FIG. 9

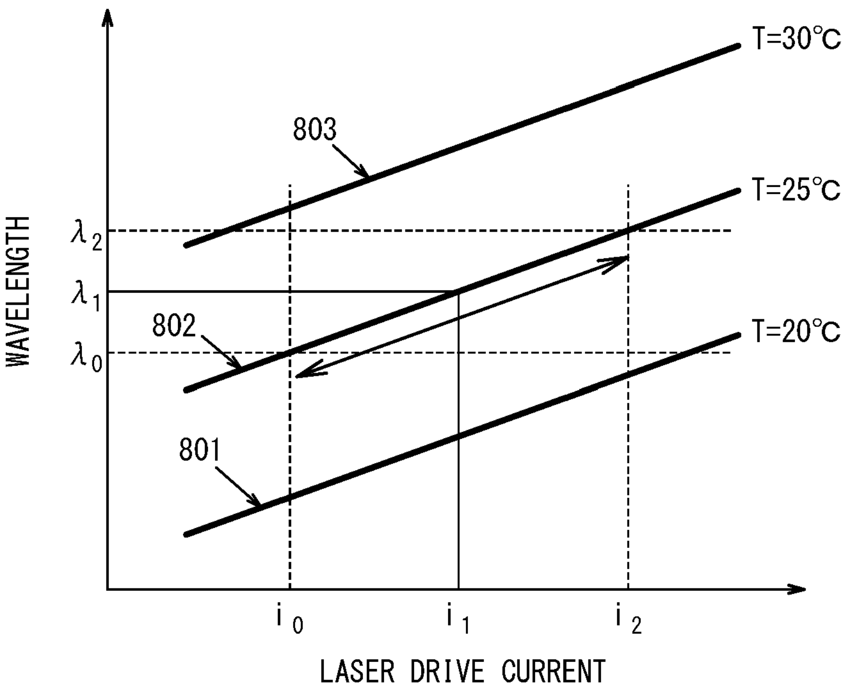
T=30°C
803
T=25°C
λ 2
WAVELENGTH
λ 1
802
T=20°C
λ 0
801
i 0
i 1
i 2
LASER DRIVE CURRENT

FIG. 10

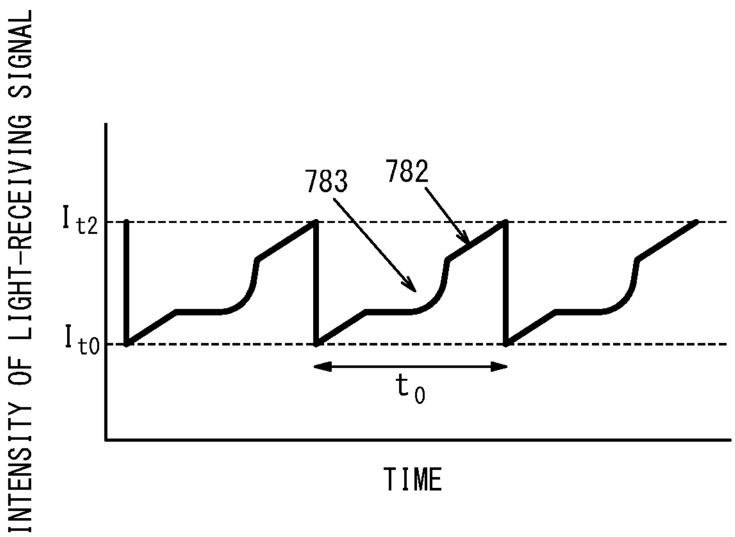

FIG. 11

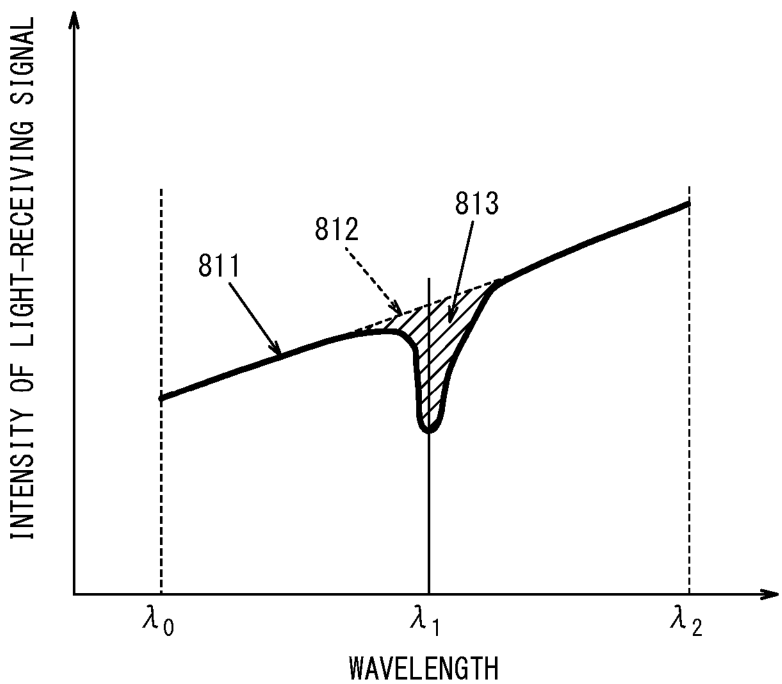
INTENSITY OF LIGHT-RECEIVING SIGNAL
811
812
813
λ0
λ1
λ2
WAVELENGTH

FIG. 12

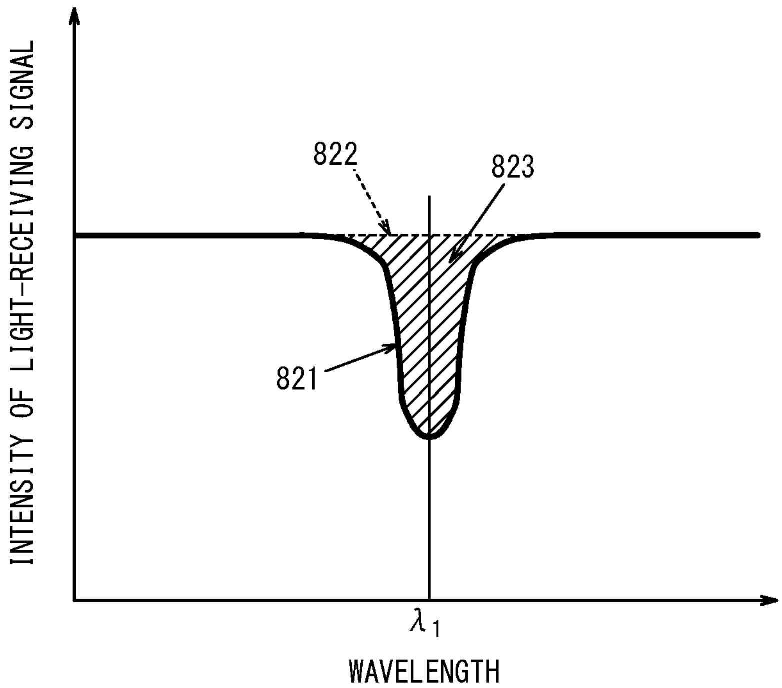
INTENSITY OF LIGHT-RECEIVING SIGNAL
822
823
821
λ1
WAVELENGTH

FIG. 13

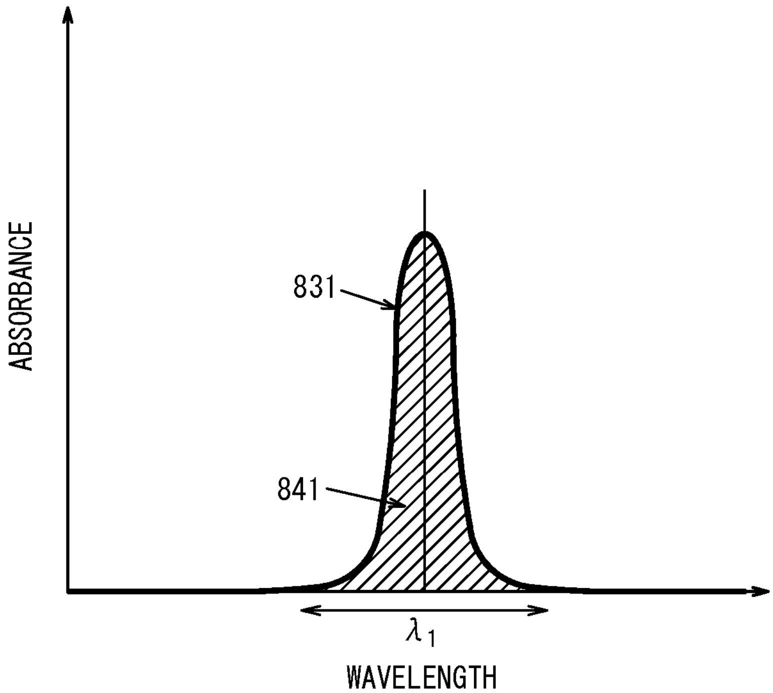

FIG. 14

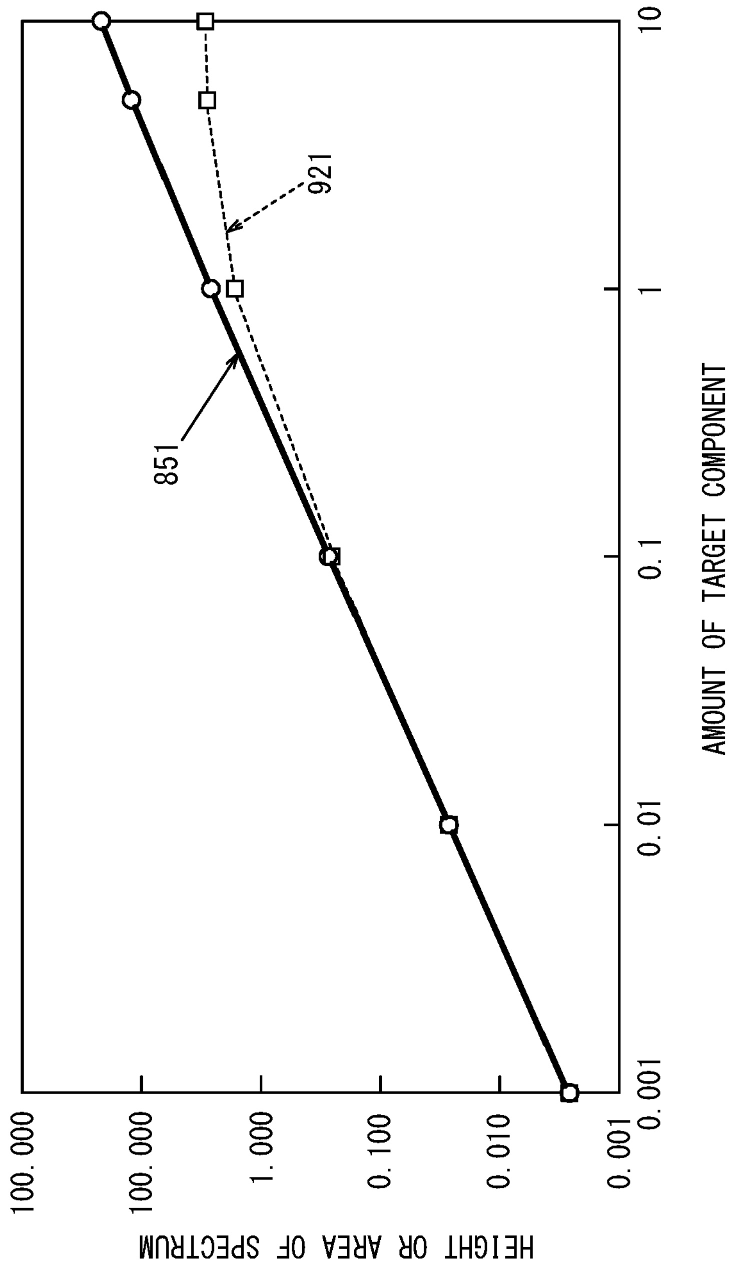

HEIGHT OR AREA OF SPECTRUM
100.000
100.000
1.000
0.100
0.010
0.001
AMOUNT OF TARGET COMPONENT
0.001
0.01
0.1
1
10
851
921

FIG. 15

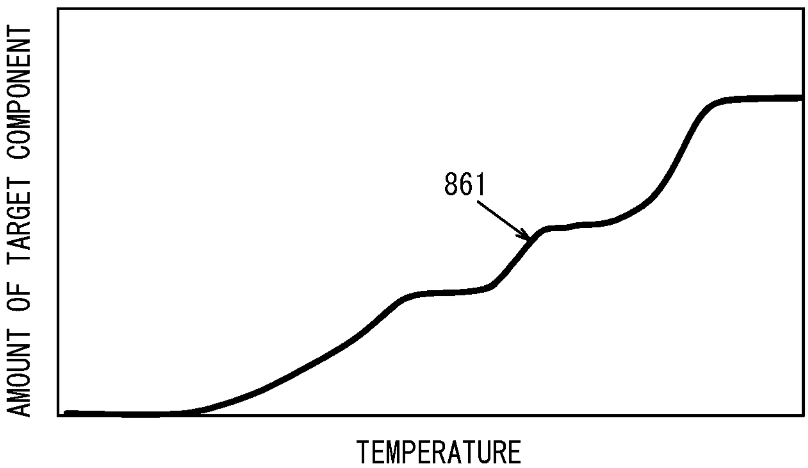

Europäisches Patentamt
European Patent Office
Office européen des brevets

# EUROPEAN SEARCH REPORT

Application Number
EP 24 19 0093

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MESHCHERINOV V V ET AL: "Lunar multichannel diode laser spectrometer DLS-L for in-situ study of samples pyrolytically evolved from regolith onboard Luna-27 mission", PROCEEDINGS OF THE SPIE, SPIE, US, vol. 11916, 15 December 2021 (2021-12-15), pages 119162O-119162O, XP060150897, ISSN: 0277-786X, DOI: 10.1117/12.2602144 ISBN: 978-1-5106-5738-0 * the whole document * ----- | 1-10 | INV. G01N21/3504 G01N21/39 ADD. G01N21/359 |
| Y | DURRY G. ET AL: "Near infrared diode laser spectroscopy of C2H2, H2O, CO2 and their isotopologues and the application to TDLAS, a tunable diode laser spectrometer for the martian PHOBOS-GRUNT space mission", APPLIED PHYSICS B, vol. 99, no. 1-2, 2 March 2010 (2010-03-02), pages 339-351, XP093232038, Berlin/Heidelberg ISSN: 0946-2171, DOI: 10.1007/s00340-010-3924-y Retrieved from the Internet: URL:http://link.springer.com/article/10.1007/s00340-010-3924-y/fulltext.html> * the whole document * ----- | 1-10 | TECHNICAL FIELDS SEARCHED (IPC): G01N |

-/--

The present search report has been drawn up for all claims

| Place of search | Date of completion of the search | Examiner |
|---|---|---|
| Munich | 6 December 2024 | Riblet, Philippe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2
EPO FORM 1503 03.82 (P04C01)

Europäisches Patentamt
European Patent Office
Office européen des brevets

# EUROPEAN SEARCH REPORT

Application Number
EP 24 19 0093

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ZENINARI V ET AL: "Laboratory spectroscopic calibration of infrared tunable laser spectrometers for the in situ sensing of the Earth and Martian atmospheres", APPLIED PHYSICS B ; LASERS AND OPTICS, SPRINGER, BERLIN, DE, vol. 85, no. 2-3, 28 June 2006 (2006-06-28), pages 265-272, XP019442385, ISSN: 1432-0649, DOI: 10.1007/S00340-006-2331-X * Section 2.; the whole document * ----- | 1-10 | |
| X | STEFAN C. SCHNEID ET AL: "Non-invasive product temperature determination during primary drying using tunable diode laser absorption spectroscopy", JOURNAL OF PHARMACEUTICAL SCIENCES, vol. 98, no. 9, September 2009 (2009-09), pages 3406-3418, XP055075052, ISSN: 0022-3549, DOI: 10.1002/jps.21522 * abstract * * page 3408, column 2 * ----- | 1,3,4,6, 8,9 | TECHNICAL FIELDS SEARCHED (IPC) |

The present search report has been drawn up for all claims

2

| Place of search | Date of completion of the search | Examiner |
|---|---|---|
| Munich | 6 December 2024 | Riblet, Philippe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 2023123851 A

### Non-patent literature cited in the description

- Lunar Polar Exploration Mission: Study on water resource observation. **SHIMOMURA TAKUMA**. Proceedings of the 66th Space Sciences and Technology Conference. The Japan Society for Aeronautical and Space Sciences, 2022